# EUROPÄISCHE PATENTANMELDUNG

(11) **EP 2 409 759 A1**
(43) Veröffentlichungstag der Anmeldung: **25.01.2012**
(21) Anmeldenummer: 10007661.1
(22) Anmeldetag: 23.07.2010
(51) Int. Cl.: B01J 21/04, B01J 23/58, C07C 29/56, C07C 35/12

(54) **Isomerisierungskatalysator**

(71) Anmelder: LANXESS Deutschland GmbH, 51369 Leverkusen (DE)
(72) Erfinder: Heuer, Lutz, 41542 Dormagen (DE); Zirngiebl, Eberhard, 51061 Köln (DE); Mechelhoff, Martin, 50733 Köln (DE)

(57) **Zusammenfassung**

Die vorliegende Erfindung betrifft ein Verfahren zur Herstellung von Mentholisomeren durch selektive Umlagerung von Stereoisomeren des Menthols oder Mischungen davon in Gegenwart von mit Erdalkalimetallalkoholaten dotierten oder enthaltenden, Ruthenium enthaltenden Trägerkatalysatoren sowie die Katalysatoren selbst.

## Beschreibung

Die vorliegende Erfindung betrifft ein Verfahren zur Herstellung von Mentholisomeren durch selektive Umlagerung von Stereoisomeren des Menthols oder Mischungen davon in Gegenwart von mit Erdalkalimetallalkoholaten dotierten oder enthaltenden, Ruthenium enthaltenden Trägerkatalysatoren sowie die Katalysatoren selbst.

Unter den natürlich vorkommenden cyclischen Terpenalkoholen nimmt das 1-Menthol, der Hauptbestandteil des Pfefferminzöls, aufgrund seiner kühlenden und erfrischenden Wirkung eine Sonderstellung ein. 1-Menthol findet daher Verwendung als Riech- oder Geschmackstoff und wird in der Arzneimittelindustrie eingesetzt.

Die Mentholherstellung durch katalytische Hydrierung von Verbindungen, die das Kohlenstoffgerüst des Menthans mit wenigstens einer Doppelbindung besitzen und in 3-Stellung durch Sauerstoff substituiert sind, wie beispielsweise Thymol, führt zu einem Stereomerengemisch der acht optisch aktiven Menthole: d,1-Menthol, d,1-Isomenthol, d,1- Neomenthol und d,1-Neoisomenthol. Diese acht optisch aktiven Menthole unterscheiden sich in Bezug auf ihre organoleptischen Eigenschaften. Nur 1-Menthol hat den charakteristischen Pfefferminzgeruch und die schon erwähnte erfrischende Wirkung. Es ist deshalb das wirtschaftlich bedeutenste der Menthol-Stereoisomeren. Andere Isomere des Menthol besitzen andere, für einige Anwendungen wünschenswerte organoleptische Eigenschaften. Daher ist man generell bestrebt, die Hydrierung durch geeignete Wahl der Reaktionsbedingungen und der Katalysatoren so zu führen, dass möglichst viel d,1-Menthol entsteht. Durch Auftrennung des Gemisches der stereoisomeren Menthole wird dann das d,1-Menthol als Racemat erhalten, das anschließend über in an sich bekannte Methoden in die Antipoden gespalten werden kann. Einmal aufgespaltene Stereoisomere des Menthols bieten bei Vorliegen eines selektiven Katalysators die Möglichkeit, andere Isomere des Menthol ebenfalls als optisch angereicherte oder reine Antipode zu erlangen.

Die Siedepunkte von d,1-lsomenthol (218,6°C bei 1013 hPa; 75 bis 78°C bei 3,3 hPa) und d,l-Menthol (216,5°C bei 1013 hPa; 75 bis 78°C bei 3,3 hPa) liegen sehr nahe beieinander. Die Trennleistung einer Kolonne bei der destillativen Trennung der einzelnen Menthol-Isomere wird daher insbesondere durch das Verhältnis von d.1-Menthol zu d,1-Isomenthol bestimmt. Für eine hohe Raum-Zeit-Ausbeute an d,1-Menthol bei der destillativen Trennung ist deshalb neben einem möglichst hohen d,1-Menthol-Gehalt im zu trennenden Gemisch auch ein möglichst geringer d,l-Isomenthol-Gehalt nötig. Die Ausbeute an Menthol wird also bei einer gegebenen Destillationskolonne wesentlich durch das Eingangsverhältnis von d,1-Menthol zu d,1-Isomenthol bestimmt.

Zur Herstellung von d,1-Menthol ist es bekannt, Verbindungen, die das Kohlenstoffgerüst des Menthans mit wenigstens einer Doppelbindung besitzen und in 3- Stellung durch Sauerstoff substituiert sind, wie beispielsweise Thymol, in kontinuierlichen Prozessen an festen Katalysatorschüttungen mit Wasserstoff zu hydrieren bzw. Stereoisomere des Menthols an festen Katalysatorschüttungen umzulagern.

In DE 23 14 813 A1 wird ein Verfahren zur Hydrierung von Verbindungen, die das Kohlenstoffgerüst des Menthans mit wenigstens einer Doppelbindung besitzen und in 3-Stellung durch Sauerstoff substituiert sind, an einer Schüttung eines Kobalt-Mangan-Katalysators bei Temperaturen von 170°C bis 220°C und einem Druck von über 25 bar, bevorzugt über 200 bar beschrieben. In den Beispielen wird bei Temperaturen von 180°C bis 210°C und bei Drucken über 200 bar gearbeitet und dabei ein Gemisch der acht stereoisomeren Menthole erhalten, das zu 59,5 bis 59,9 % aus dem racemischen d,1-Menthol und zu 10,6 bis 10,8 % aus d,1-Isomenthol besteht. Das Menthol-Isomenthol-Verhältnis beträgt dabei maximal 5,7. Durch Modifikation des Kobalt-Mangan-Katalysators mit Kupfer wurden Menthol-Gemische mit d,1-Menthol-Gehalten von 57,6 % und d,1- Isomenthol-Gehalten von 9,2% erreicht, was einem Menthol-Isomenthol-Verhältnis von etwa 6,3 entspricht. Die erhaltenen Gemische weisen jedoch 4 bis 5 % an unerwünschten Nebenprodukte in Form von nicht wiederverwertbaren Kohlenwasserstoffen auf.

Aus EP 0 563 611 A 1 und DE 197 18 116 A 1 ist bekannt, dass die Hydrierung von aromatischen oder teilhydrierten cyclischen Verbindungen, die das Kohlenstoffgerüst des Menthans mit wenigstens einer C=C-Doppelbindung besitzen und in 3-Stellung durch Sauerstoff substituiert sind, mit Wasserstoff an einem Festbettkatalysator durchgeführt werden kann, der Palladium, Ruthenium oder Rhodium oder ein Gemisch dieser Elemente als Aktivbestandteile und Alkalimetallhydroxide und/oder -sulfate als Promotoren jeweils aufgebracht auf einem Träger enthält, wobei der Träger mit einem Metall der Seltenen Erden und Mangan dotiert ist. In den Beispielen wurde bei Temperaturen von 180 bis 240°C und Drucken von 270 bis 300 bar gearbeitet. Dabei wurden Menthol-Gemische erhalten, die ca. 52 bis 57 % d,1-Menthol und 11,5 bis 14,8 % d,1-Isomenthol gebildet, was einem Menthol-Isomenthol-Verhältnis von 3,6 bis 4,4 entspricht.

In EP 743 296 A 1 werden Katalysatoren offenbart, die aus trägerfreien, verpressten Pulvern von Kobalt-, Mangan- und Erdalkalioxiden bzw. -hydroxiden bestehen und bei Temperaturen von 150°C bis 230°C und Drucken von 25 bis 350 bar eingesetzt werden. In den angegebenen Beispielen wird bei Temperaturen über 165°C und Drucken von mehr als 200 bar gearbeitet. Auf die Zusammensetzung der entstehenden Menthol-Gemische wird nicht eingegangen.

Die Umlagerung von Stereoisomeren des 1-Menthols ist in US 5 756 864 beschrieben: Bei Temperaturen von 200 bis 350°C und Wasserstoff-Drucken von 50 bis 350 bar, bevorzugt 100 bis 300 bar wird d-Menthol in einem kontinuierlichen Prozess an einem Katalysator racemisiert und isomerisiert, wobei der Katalysator aus trägerfreien, gepressten Pulvern von Nickel-, Mangan- und Erdalkalihydroxiden bzw. -oxiden besteht. Dabei wurden Menthol-Gemische erhalten, die maximal zu 59,8 % aus d,l- Menthol bestanden.

Aus US 2 843 636 ist bekannt, die Isomerisierung von Stereoisomeren des Menthols zu d,l-Menthol mit Wasserstoff in Gegenwart eines Hydrierkatalysators aus der Gruppe Kupferchromit, Kobalt und Nickel bei 260 bis 280°C und 500 bis 1300 p.s.i.g. (34 bis 90 bar) in Autoklaven durchzuführen. Die entstehenden Gemische wiesen neben ca. 10 bis 12 % d,1-Isomenthol einen d,l-Menthol-Gehalt von 60 bis 64 % auf.

In DE 198 53 562 A wird eine Niederdruck-Hydrierung von Thymol an einer stationären Katalysatorschüttung beschrieben, die einen Temperaturgradienten aufweist: Die ersten zwei von fünf in Serie geschalteten Reaktorrohren werden auf 180°C temperiert, die hinteren drei Reaktorrohre auf 80 bis 90°C. Mit einem Katalysator, der auf einem Träger, der mit einem Metall der Seltenen Erden und mit Mangan dotiert ist, Ruthenium als Aktivbestandteil und Alkalimetallhydroxide als Promotoren enthält, konnte bei einem Druck von 3 bar ein Menthol-Isomerengemisch erhalten werden, das 64,4 Gew-% Menthol und 12,1 % Isomenthol enthielt, was einem Menthol-Isomenthol-Verhältnis von 5,3 entspricht. Die Isomerisierung einer wasserstoffgesättigten Mischung aus d,1-Neomenthol, d,1-Isomenthol und d,1-Menthol lieferte bei Normaldruck ein Isomerengemisch mit einer Zusammensetzung von 65,3 % d,1- Menthol und 12,1 % Isomenthol. Bei diesem Niederdruckverfahren lassen sich hohe Menthol-Gehalte von ca. 65 % erzielen. Das Menthol-Isomenthol-Verhältnis beträgt jedoch maximal 5,4.

In der DE 100 23 283 A wird nun ein verbessertes Verfahren beschrieben, in welchem Isomerengemische, die typischerweise etwa 55 % d,1-Menthol aufweisen, durch Isomerisierung mit einfachen Ruthenium-Trägerkatalysatoren Menthol-reichere Gemische herstellen kann, die bis zu 67,3 % d,1-Menthol und lediglich 8,2 % d,1-Isomenthol d.h. ein Menthol-Isomenthol-Verhältnis von bis zu 8,1 aufweisen. Des weiteren ist aus DE 100 23 283 A bekannt, dass die Katalysatoren mit Alkoholaten, Oxiden und Hydroxiden der Alkali- oder Erdalkalimetalle regeneriert werden können.

Allen bekannten Verfahren ist demnach gemeinsam, dass sie mindestens 8,2 % d,1-Isomethol ergeben und maximale Menthol-Isomenthol-Verhältnisse von 8,1 erlauben.

Aufgabe der Erfindung war es deshalb, ein selektives und technisch einfaches Verfahren für die Herstellung von d,1-Menthol zu finden, das nur geringe Mengen an d,1-Isomenthol ergibt und hohe Menthol-Isomenthol-Verhältnisse erlaubt, wobei gleichzeitig die Bildung unerwünschter Nebenprodukte weitgehend vermieden wird.

In einem weiteren Aspekt bestand die Aufgabe darin, diese Umwandlung von im wesentlichen reinen Stereoisomeren des Menthols möglichst so selektiv zu gestalten, dass sie katalytisch zumindest teilsweise in ein anderes Stereoisomer überführt werden können, ohne das die optische Aktivität verloren geht.

Vorab sei angemerkt, dass es unter den nachstehend genannten Prozentangaben wie auch im Stand der Technik üblich Flächenprozent zu verstehen sind, die sich bei der gaschromatographischen Analyse des Produktgemisches ergeben. Unter Menthol-Isomenthol-Verhältnis ist folglich das Verhältnis von Flächenprozent (GC) d,1-Menthol zu Flächenprozent (GC) d,1-Isomenthol zu verstehen.

Es wurde nun ein Katalysator gefunden, der Ruthenium aufgebracht auf einem Trägermaterial enthält, wobei das Trägermaterial Aluminiumoxid ist und der dadurch gekennzeichnet ist,
- dass er zumindest ein Erdalkalimetallalkoxylat enthält oder
- durch Umsetzung eines Katalysators, der Ruthenium aufgebracht auf einem Trägermaterial enthält, wobei das Trägermaterial Aluminiumoxid ist, mit zumindest einem Erdalkalimetallalkoxylat erhältlich ist.

Weiterer Gegenstand der Erfindung ist ein Verfahren zur Isomerisierung von Stereoisomeren des Menthols oder Gemischen solcher Stereoisomeren in Gegenwart des vorgenannten Katalysators.

Der Gegenstand der Erfindung umfasst nicht nur die offenbarten allgemeinen oder bevorzugten Ausführungsformen für einzelne Parameter oder Verbindungen, sondern auch jede denkbare Kombination davon.

Das als Trägermaterial eingesetzte Aluminiumoxid kann in allen bekannten Modifikationen, bevorzugt in der y-Modifikation eingesetzt werden. Vorteilhaft weist das als Trägermaterial eingesetzte Aluminiumoxid eine BET-Oberfläche von mindestens 100 m²/g, bevorzugt mindestens 160 m²/g, besonders bevorzugt mindestens 180 m²/g auf. Besonders bevorzugt ist Aluminiumoxid, das zusätzlich einem hohen Anteil an makroporösen Poren mit einem Porendurchmesser von mindestens 50 nm aufweist und ein Porenvolumen von mindestens 300 mm³/g, bevorzugt mindestens 600 mm³/g besitzt. Als geeignete Trägermaterialien seien beispielhaft die kommerziell erhältlichen Aluminiumoxide SPH 1515, SPH 531, SPH 501 der Firma Rhodia, D 10-10 der Firma BASF und SA 6176 der Firma Norton genannt.

Das Trägermaterial kann beispielsweise in Form von Pulver mit Korngrößen von 0,001 bis 0,1 mm, gebrochenem und gesiebtem Material mit Korngrößen zwischen 0,05 bis 5 mm oder in Formkörpern wie Strangpresslingen, Extrudaten, Pillen, Kugeln oder Granulaten mit Durchmessern von 0,2 bis 30 mm eingesetzt werden.

Zur Herstellung der Katalysatoren kann man beispielsweise so vorgehen, dass man auf eines der genannten Trägermaterialien zunächst Ruthenium und gegebenenfalls eines oder mehrere weitere Metalle der 8. Nebengruppe des Periodensystems und/oder Zinn und/oder Zink aufträgt. Das Auftragen kann durch Behandeln, beispielsweise Tränken oder Besprühen des Trägermaterials mit Lösungen von Salzen der Metalle geschehen. Dazu werden beispielsweise die Chloride, Acetate und/oder Nitrate eingesetzt. Dieses Aufbringen der Metalle kann in einem Schritt mit gelösten Mischungen der Salze oder nacheinander mit den Lösungen der Einzelverbindungen erfolgen. Nach jedem Auftrag kann der Katalysator getrocknet werden.

Die Menge an Ruthenium bzw. an Rutheniumverbindung zur Herstellung des erfindungsgemäßen Katalysators beträgt bzw. wird so gewählt dass der Katalysator bezogen und berechnet auf Ruthenium beispielsweise 0,1 bis 35 Gew. -%, vorzugsweise 1 bis 10 Gew.-% enthält.

Ein in der genannten Weise hergestellter Katalysator wird beispielsweise durch Behandlung mit Wasserstoff oder Wasserstoff enthaltenden Gasen wie beispielsweise Stickstoff mit einem Geghalt an Wasserstoff von 0,5 bis 10 vorzugsweise 0,5 bis Vol-% Wasserstoff beispielsweise bei einer Temperatur von 20 bis 400°C, bevorzugt 30 bis 250°C reduziert. Die Reduktion kann auch mit anderen Reduktionsmitteln wie z. B. Hydrazin erfolgen. Der reduzierte Katalysator wird vorzugsweise im Anschluss gewaschen um gegebenenfalls noch anhaftende Salze zu entfernen.

Das aufgebrachte Metall kann beispielsweise auch auf dem Träger fixiert werden, indem man den mit Ruthenium und gegebenenfalls weiteren Metallen getränkten Träger mit einer Lösung von basischen Salzen, wie beispielsweise Alkalimetall- oder Erdalkalimetallhydroxiden oder -oxiden wie beispielsweise Natriumhydroxid oder Kaliumhydroxid behandelt, wobei das Metall als Oxid oder Hydroxid ausfällt. Ist das Metall auf dem Träger fixiert, können die Reduktion und das Auswaschen löslicher Bestandteile in beliebiger Reihenfolge durchgeführt werden. Bevorzugt werden zunächst die löslichen Bestandteile ausgewaschen und der Katalysator dann reduziert. Die Reduktion kann auch in situ bei der Durchführung des erfindungsgemäßen Verfahrens erfolgen.

Nach jedem Tränkschritt mit Metallsalz-Lösung oder basischen Salzen oder nach Waschungen kann ein Trocknungsschritt durchgeführt werden. Es ist aber auch möglich den Träger ohne Trocknung im nächsten Herstellschritt einzusetzen.

Der so hergestellte Katalysator kann dann, in einer Ausführungsform mit Erdalkalimetallhydroxiden wie vorzugsweise Bariumhydroxid beispielsweise bei 20°C bis zum Siedepunkt der Lösung dotiert werden, wobei die Dotierung vorzugsweise so erfolgt, dass der Katalysator gleichmäßig mit der Lösung von Erdalkalimetallhydroxiden wie vorzugsweise Bariumhydroxid in Kontakt gerät. Das hierzu notwendige Wasser zieht dabei in die Katalysatorforrnkörper ein. Das Besprühen kann unter Umgebungsbedingungen erfolgen oder unter Inertgas.

Ein so hergestellter Katalysator kann ebenso wie ein nicht dotierter Katalysator im Reaktionssystem reduziert und zur Isomerisierung eingesetzt werden (Beispiel 1, Absetzbeispiel zu DE 198 53 562 und DE 100 23 283).

Erfindungsgemäß wird eine weitere Steigerung der Aktivität und Seleketivität erreicht werden, wenn der bereits mit Erdalkalimetallen dotierte Katalysator oder ein nicht mit Erdalkalimetallen dotierter Katalysator durch Inkontaktbringen mit zumindest einem Erdalkalimetallalkoxylat (weiter) dotiert wird.

Zu diesem Zweck können beispielsweise Erdalkalimetallalkoxylate der Formel (I) eingesetzt werden,

(R-O)₂M (I)

in der
- R: jeweils unabhängig, vorzugsweise identisch für einen primären, sekundären oder tertiären, cyclischen oder acylischen, verzweigten oder unverzweigten Cl bis C20-Alkylrest steht, der gegebenenfalls durch Aryl, C₁-C₄-Alkoxyl oder C₆ bis C₁₄-Aryloxy weiter substituiert sein kann und vorzugsweise für Methyl, Ethyl, n-Propyl, Isopropyl, n-Butyl, sec.-Butyl, tert. Butyl, n-Pentyl, Neopentyl, n-Hexyl, Cyclohexyl oder die stereoisomeren Menthylreste und
- M: für Calcium, Strontium oder Barium, vorzugsweise Barium steht.

Die bevorzugte Bariumalkoxylate können beispielsweise durch Umsetzung von Bariumperchlorat mit den entsprechenden Kaliumalkoxylaten vorzugsweise gelöst im gleichen oder einem anderen Alkohol erhalten werden, wobei sich schwerlöslichen Kaliumperchlorat bildet, welches aus den Reaktionslösungen beispielsweise durch Filtration leicht entfernt werden kann.

Bariummentholates sind beispielsweise auch erhältlich durch Versetzen von Bariumethoxid oder Bariumisopropoxid mit einem Überschuß an Menthol-Stereoisomeren und längerem Stehenlassen oder Erwärmen.

Besonders bevorzugt finden Bariumethoxid, 10% w/v in Ethanol, Bariumisopropoxid als feste Substanz, gelöst in Mentholisomeren oder Bariumisopropoxid, 20% w/v in Isopropanol Verwendung.

Die Dotierung kann beispielsweise derart erfolgen, dass die Erdalkalimetallalkoxylate beispielsweise in Lösung eines von Menthol verschiedenen Alkohols in den Feed-Strom der als Edukte eingesetzten Menthole wie beispielsweise der Stereoisomerenmischungen der Menthole zugesetzt werden, und der Katalysator auf diese Weise weiter oder erstmals dotiert wird.

Dieses Verfahren hat den Vorteil, dass beispielsweise mit Erdalkalimetallhydroxiden-dotierte Katalysatoren nicht der Luft ausgesetzt werden, an der mit dem Kohlendioxid der Luft Erdalkalimetallcarbonate gebildet werden.

Die Menge des Erdalkalimetallalkoxylates das zur Herstellung des erfindungsgemäßen Katalysators eingesetzt wird beträgt oder wird so gewählt dass das molare Verhältnis von Ruthenium zu Erdalkalimetall beispielsweise 30:1 bis 1:30 vorzugsweise 10:1 bis 1:10 beträgt.

Der erfindungsgemäß Katalysator eignen sich insbesondere zur Herstellung von d,1-Menthol durch katalytische Isomerisierung von Stereoisomeren des Menthols oder Mischungen dieser Stereoisomere sowie weiterhin zur stereoselektiven Umwandlung von
(+)-Menthol (d-Menthol) zu (-)-Neomenthol,
(-)-Menthol (1-Menthol) zu (+)-Neomenthol,
(+)-Isomenthol zu (-)-Neoisomenthol oder
(-)-Isomenthol zu (+)-Neoisomenthol.

Daher ist von der Erfindung auch die Verwendung der erfindungsgemäßen Katalysatoren zur Isomerisierung von Stereoisomeren des Menthols oder Mischungen dieser Stereoisomere umfasst.

Als Edukt können entweder die im Wesentlichen reinen Isomere des Menthols eingesetzt werden oder beliebige Gemische von Stereoisomeren davon.

Im Wesentlichen reine Stereoisomere bedeutet dabei einen Gehalt des jeweiligen Stereoisomers des Menthols bezogen auf den Gesamtgehalt an den 8 isomeren Mentholen von 95 % oder mehr vorzugsweise 98 % oder mehr besonders bevorzugt 99 % oder mehr.

Gemische von Stereoisomeren fallen beispielsweise bei der Racemisierung von optisch aktiven Mentholen an oder bleiben bei der destillativen Abtrennung von d,1-Menthol aus einem Stereoisomerengemisch zurück. Ferner können beispielsweise Menthol-Isomerengemische eingesetzt werden, die bei der Hydrierung von cyclischen Verbindungen entstehen, die das Kohlenstoffgerüst des Menthans mit wenigstens einer Doppelbindung besitzen und in 3-Stellung durch Sauerstoff substituiert sind, wie zum Beispiel Thymol, Menthon oder Isomenthon.

Solche Ausgangsmischungen enthalten beispielsweise zwischen:
0 und 100 %, vorzugsweise 30 bis 70 % d,1-Menthol oder D- oder L-Menthol
0 und 100 %, vorzugsweise 2 bis 30 % d,1-Isomenthol oder (+)- oder (-)-Isomenthol
0 und 100 %, vorzugsweise 10 bis 99 % d,1- Neomenthol oder (+) oder (-) Neomenthol
0 und 100 %, vorzugsweise 0,1 bis 70 % d,1-Neoisomentholoder (+)- oder (-)-Neoisomenthol,
wobei die Massgabe gilt, dass mindestens zwei Stereoisomere in der Mischung vorhanden sein müssen, sich die Summe der acht vorgenannten Verbindungen zu 90 bis 100 %, vorzugsweise zu 95 bis 100 % der Ausgangsmischung addieren und der maximale Gehalt eines Stereoisomeren des Menthols in der Mischung bezogen auf den Gesamtgehalt an den 8 isomeren Mentholen unter 95 % beträgt.

Bevorzugte Mischungen sind solche, die
30 bis 70 % d,1-Menthol oder D- oder L-Menthol
2 bis 30 % d,1-Isomenthol oder (+)- oder (-)-Isomenthol
10 bis 99 % d,1- Neomenthol oder (+) oder (-) Neomenthol und
0,1 bis 70 % d,1-Neoisomenthol oder (+)- oder (-)-Neoisomenthol,
enthalten wobei die Massgabe gilt, dass sich die Summe der acht vorgenannten Verbindungen zu 90 bis 100 %, vorzugsweise zu 95 bis 100 % der Ausgangsmischung addieren.

Besonders bevorzugte Mischungen sind solche, die
30 bis 70 % d,1-Menthol
2 bis 30 % d,1-Isomenthol
10 bis 99 % d,1- Neomenthol und
0,1 bis 70 % d,1-Neoisomenthol
enthalten wobei die Massgabe gilt, dass sich die Summe der vier vorgenannten racemischen Enantiomerenpaare zu 90 bis 100 %, vorzugsweise zu 95 bis 100 % der Ausgangsmischung addieren.

Die Isomerisierung kann beispielsweise in an sich bekannten Reaktoren und entweder diskontinuierlich oder kontinuierlich durchgeführt werden, wobei eine kontinuierliche Durchführung bevorzugt ist.

Bei diskontinuierlichen Verfahren liegt die Katalysatorbelastung beispielsweise bei 0,0001 bis 10 kg Katalysator pro kg Edukt, bevorzugt bei 0,01 bis 0,1 kg Katalysator pro kg Edukt.

Bei kontinuierlichen Verfahren liegt die Katalysatorbelastung beispielsweise bei 0,005 bis 5 kg Ausgangsprodukt pro Liter Katalysator und Stunde [kg/1*h], bevorzugt bei 0,03 bis 2 kg/I*h, besonders bevorzugt bei 0,06 bis 1,0 kg/I*h.

Mit steigender Katalysatorbelastung erhöht sich die Raum-Zeit-Ausbeute für das erfindungsgemäße Verfahren. Allerdings sinkt gleichzeitig auch das und das Menthol-Isomenthol-Verhältnis, wobei der Grad der Abnahme stark von der gewählten Reaktionstemperatur abhängt.

Die maximale Katalysatorbelastung bei einer gegebenen Reaktionstemperatur bei der noch das gewünschte Ergebnis erzielt wird, ist für den Fachmann in wenigen Versuchen leicht bestimmbar. Das erfindungsgemäße Verfahren kann z. B. im Rührkessel, als Rieselphase, in der Sumpfphase mit aufgeschlämmten Katalysator, als Blasensäule oder an einer stationären Katalysatorschüttung durchgeführt werden. Bevorzugt wird das erfindungsgemäße Verfahren in Reaktoren mit stationären Katalysatorschüttungen in der Sumpfphase durchgeführt.

Der Reaktor kann dabei beispielsweise einem Reaktor zur Hydrierung von cyclischen Verbindungen, die das Kohlenstoffgerüst des Menthans mit wenigstens einer Doppelbindung besitzen und in 3-Stellung durch Sauerstoff substituiert sind, oder einem Reaktor zur Racemisierung/Isomerisierung von d-Menthol oder anderen Isomeren des 1-Menthols oder einer Trennvorrichtung wie insbesondere einer Destillations- oder Rektifikationsanlage für Stereoisomerengemische des Menthols nachgeschaltet sein.

Der Reaktor, in dem das erfindungsgemäße Verfahren durchgeführt wird, kann beispielsweise auch zwischen einem Reaktor zur Hydrierung, Isomerisierung oder Racemisierung und einer nachfolgenden Trennvorrichtung wie insbesondere einer Destillations- oder Rektifikationsanlage geschaltet sein.

Gegebenenfalls können verschiedene Produktströme zum Beispiel aus der Hydrierung und/oder der Trennung vermischt als Edukt im erfindungsgemäßen Verfahren eingesetzt werden. Der Reaktor zur Isomerisierung kann aber gegebenenfalls auch isoliert betrieben werden.

In einer Ausführungsform der Verschaltung eines solchen mit dem erfindungsgemäßen Katalysator gefüllten Reaktors ist vorteilhaft, einen bereits rektifizierten Mentholstrom, der arm an d,1-Menthol ist als Edukt zu verwenden, wobei ganz besonders bevorzugt ein Neomenthol-haltiger Strom bevorzugt wird.

Das nach dem erfindungsgemäßen Verfahren hergestellte d,1-Menthol-haltige Isomerengemisch kann zur Isolierung von reinem d,1-Menthol in an sich bekannter Weise aufgetrennt werden beispielsweise durch Destillation oder Rektifikation.

Das erfindungsgemäße Verfahren kann in Gegenwart von Lösungsmitteln durchgeführt werden. Bevorzugt ist jedoch eine lösungsmittelfreie Durchführung.

Das erfindungsgemäße Verfahren kann beispielsweise bei einem Gesamtdruck von 25 hPa bis 30 MPa, vorzugsweise 50 hPa bis 5 MPa, besonders bevorzugt bei 100 hPa bis 2 MPa und ganz besonders bevorzugt bei 1000 hPa bis 1 MPa durchgeführt werden.

Das erfindungsgemäße Verfahren kann auch unter Zusatz oder ohne Zusatz von Wasserstoff betrieben werden:
Bei Zusatz von Wasserstoff wird beispielsweise die flüssige Phase vor dem Eintritt in den Reaktor mit Wasserstoff gesättigt, oder gasförmiger Wasserstoff zusammen mit dem Edukt in den Reaktor geleitet, so dass ein Wasserstoffpartialdruck zwischen 1 hPa bis 30 MPa, bevorzugt zwischen 10 hPa und 5 MPa , besonders bevorzugt zwischen 10 hPa und 1 MPa eingestellt wird.

Bevorzugt wird jedoch bei der Durchführung des erfindungsgemäßen Verfahrens kein Wasserstoff zugesetzt, so dass allenfalls der Wasserstoff zugegen ist, der gegebenenfalls über die verwendeten Edukte in den Reaktor eingetragen wird.

Das erfindungsgemäße Verfahren wird beispielsweise bei Temperaturen von 30 bis 170°C, bevorzugt bei Temperaturen von 50 bis 150°C, besonders bevorzugt bei Temperaturen von 70 bis 130°C und besonders bevorzugt 75 bis 115°C durchgeführt.

Der Vorteil der vorliegenden Erfindung ist insbesondere darin zu sehen, dass das erfindungsgemäße Verfahren unter Einsatz des erfindungsgemäßen Katalysators die hochselektive Isomerisierung von Stereoisomeren des Menthols erlaubt , womit die die anschließende Aufarbeitung ganz besonders effizient gestaltet werden kann. Wird von reinen, technisch verfügbaren Ausgangsmaterialien wie d-Menthol oder 1-Menthol oder Neomenthol ausgegangen, sind Menthol-Isomentholverhältnisse von über 400 erreichbar.

Die nachstehenden Beispiele sollen die Erfindung erläutern, ohne jedoch ihren Gegenstand darauf zu beschränken.

### Beispiele

### Beispiel 1: Herstellung des Katalysators

1000 g (ca. 2,24 1) eines handelsüblichen γ-Al₂O₃ mit einer BET-Oberfläche von ca. 255 m²/g (SA 6176 der Fa. Norton, Extrudat mit Teilchendurchmesser 1/16" (ca. 1,6 mm), Schüttdichte ca. 0,44 kg/l) wurde in einem Großrotationsverdampfer (10 1 Kolben) vorgelegt, mit einer wässrigen Lösung von 300 g Ruthenium(III)-chlorid (käufliche Lösung mit 20 Gew.-% Ru, 60 g Ru) in 1153 g destilliertem Wasser versetzt und für 10 Minuten rotiert (ca. 16 UpM (Umdrehungen pro Minute)). Das Lösungsmittel wurde bei 90°C und 10 mbar abdestilliert. Der Katalysator wurde im Wasserstoffstrom bei 250°C reduziert. Anschließend wurde der Katalysator solange mit destilliertem Wasser gewaschen, bis das Waschwasser Chlorid-frei war. Danach wurde der Katalysator im Rotationsverdampfer getrocknet (90°C, 10 mbar).

2000 ml (Schüttdichte ca. 0,5 kg/I) des so erhaltenen Katalysators wurden dann mit einer Lösung aus 50 g Bariumhydroxid-Octahydrat in 150 ml destilliertem Wasser bei 75 bis 90°C besprüht, so dass sich ein gleichförmiger weißer Niederschlag auf den zuvor schwarzen Katalysatorformkörpern bildete. Der Katalysator erwärmte sich dabei geringfügig.

Dieser Katalysator kam zum Vergleich unter den in Tabelle 1 genannten Bedingungen zum Einsatz.

### Beispiel 2: Dotierung

Der Katalysator gemäß Beispiel 1 wurde dann durch Bariumethoxylat-Lösung, 80 ml 10 Gew-%ig in Ethanol, eingegeben in 500 ml Mentholisomere als Feed-Strom während des Einsatzes dotiert. So wird ein hochaktiver Katalysator erhalten, der die verbesserten Ergebnisse zeigt, die in Tabellen 2, 3, 4 und 5 gezeigt sind.

### Beispiel 3: Versuchsanlage

Die Versuchsanlage bestand aus einer thermostatisierten Reaktorrohren von jeweils 1 m Länge und 45 mm Innendurchmesser. Die Beheizung der Reaktoren erfolgt durch Thermostate. Die Reaktorrohre wurden jeweils mit ca. 1500 ml des Katalysators aus Beispiel 1 gefüllt Das eingesetzte Menthol-Isomerengemisch ist der unten stehenden Tabelle zu entnehmen und wurde mittels einer Membranpumpe kontinuierlich in den Rohrreaktor gefördert. Das Edukt wurde von oben (Rieselphase) oder wahlweise von unten (Sumpfphase) durch die Rohrreaktore geleitet. Wasserstoff wurde durch Sättigung des Eduktes bei 0,6 bis 1,2 MPa zugegeben.

### Ergebnisse

**Tabelle 1:**

| Ausgangszusammensetzung: 29,0 % NM, 2,3 % NIM, 55,7 % M, 12,3 % IM | | | | | | | |
|---|---|---|---|---|---|---|---|
| **T [°C]** | **Eintrag V [g/h]** | **Produktanalyse** | | | | | **M/IM*** |
| | | **NM ?** | **NIM** | **M** | **IM** | **Summe Menthole** | |
| 101 | 97 | 27,266 | 0,871 | 63,296 | 7,990 | 99,423 | 7,9 |
| 101 | 103 | 28,696 | 0,91 | 62,129 | 7,627 | 99,363 | 8,1 |
| 101 | 103 | 28,626 | 0,922 | 62,002 | 7,772 | 99,322 | 8,0 |
| 101 | 157 | 28,914 | 1,14 | 60,293 | 9,144 | 99,49 | 6,6 |
| 101 | 157 | 28,677 | 1,178 | 60,156 | 9,516 | 99,526 | 6,3 |
| 101 | 150 | 28,969 | 1,164 | 60,123 | 9,267 | 99,522 | 6,5 |
| 101 | 200 | 28,578 | 1,449 | 58,187 | 11,207 | 99,42 | 5,2 |
| 101 | 199 | 28,527 | 1,456 | 58,145 | 11,318 | 99,447 | 5,1 |
| 105 | 199 | 29,220 | 1,295 | 59,065 | 9,815 | 99,305 | 6,0 |
| 110 | 209 | 29,768 | 1,176 | 59,631 | 8,631 | 99,228 | 6,9 |
| 115 | 205 | 30,015 | 1,132 | 59,720 | 8,119 | 98,987 | 7,4 |
| 115 | 207 | 29,947 | 1,130 | 59,835 | 8,151 | 99,063 | 7,3 |
| 120 | 214 | 30,319 | 1,158 | 59,252 | 8,061 | 97,632 | 7,4 |
| 120 | 206 | 30,064 | 1,155 | 59,416 | 8,068 | 98,704 | 7,4 |
| 130 | 201 | 29,593 | 1,231 | 58,206 | 8,339 | 97,369 | 7,0 |
| 130 | 199 | 29,888 | 1,232 | 58,007 | 8,234 | 97,361 | 7,0 |
| 120 | 248 | 29,528 | 1,133 | 60,006 | 8,254 | 98,920 | 7,3 |
| 120 | 249 | 29,517 | 1,132 | 60,010 | 8,261 | 97,787 | 7,3 |

**Tabelle 2**

| Ausgangszusammensetzung: 85,5 % NM, 3,85 % NIM, 6,65 % M, 12,3% 0,97 IM | | | | | | | |
|---|---|---|---|---|---|---|---|
| **T[°C]** | **Eintrag V [g/h]** | **Produktanalyse** | | | | | **M/IM^{*}** |
| | | **NM** | **NIM** | **M** | **IM** | **Summe Menthole** | |
| 115 | 353 | 30,172 | 1,001 | 60,895 | 7,266 | 99,335 | 8,4 |
| 115 | 357 | 30,129 | 0,991 | 61,008 | 7,245 | 99,373 | 8,4 |
| 125 | 352 | 29,856 | 1,180 | 59,633 | 8,209 | 98,698 | 7,3 |
| 114 | 349 | 30,325 | 0,979 | 60,962 | 7,078 | 99,344 | 8,6 |
| 114 | 356 | 30,281 | 0,962 | 61,135 | 6,988 | 98,404 | 8,7 |
| 114 | 355 | 29,639 | 0,934 | 61,688 | 7,111 | 99,372 | 8,7 |
| 111 | 353 | 29,317 | 0,847 | 62,655 | 6,679 | 99,498 | 9,4 |
| 111 | 352 | 29,194 | 0,834 | 62,764 | 6,638 | 99,430 | 9,5 |
| 111 | 362 | 31,777 | 0,897 | 60,655 | 6,069 | 99,399 | 10,0 |
| 111 | 357 | 30,270 | 0,815 | 62,305 | 6,097 | 99,487 | 10,2 |
| 109 | 349 | 30,057 | 0,801 | 62,537 | 6,119 | 99,513 | 10,2 |
| 109 | 351 | 30,119 | 0,806 | 62,445 | 6,135 | 99,505 | 10,2 |
| 106 | 347 | 29,313 | 0,719 | 63,602 | 5,910 | 99,545 | 10,8 |
| 106 | 349 | 29,167 | 0,707 | 63,799 | 5,854 | 99,527 | 10,9 |
| 106 | 349 | 29,088 | 0,701 | 63,861 | 5,854 | 99,504 | 10,9 |
| 103 | 349 | 28,540 | 0,622 | 64,905 | 5,477 | 99,544 | 11,9 |
| 103 | 354 | 28,451 | 0,616 | 64,985 | 5,496 | 99,547 | 11,8 |
| 100 | 354 | 27,982 | 0,558 | 65,795 | 5,240 | 99,574 | 12,6 |
| 100 | 344 | 28,074 | 0,558 | 65,747 | 5,191 | 99,571 | 12,7 |
| 97 | 344 | 28,260 | 0,512 | 65,679 | 5,106 | 99,557 | 12,9 |
| 100 | 349 | 27,728 | 0,526 | 66,208 | 5,13 | 99,592 | 12,9 |
| 100 | 347 | 27,590 | 0,519 | 66,339 | 5,188 | 99,636 | 12,8 |
| 100 | 171 | 29,148 | 0,568 | 64,911 | 4,960 | 99,587 | 13,1 |
| 100 | 175 | 29,557 | 0,627 | 64,145 | 5,261 | 99,590 | 12,2 |
| 100 | 164 | 28,553 | 0,600 | 65,108 | 5,390 | 99,651 | 12,1 |
| 95 | 175 | 27,987 | 0,540 | 65,885 | 5,182 | 99,594 | 12,7 |
| 95 | 168 | 27,847 | 0,521 | 66,116 | 5,090 | 99,575 | 13,0 |

**Tabelle 3**

| Ausgangszusammensetzung: 99,7 % D-Menthol | | | | | | | |
|---|---|---|---|---|---|---|---|
| **T [°C]** | **Eintrag V [g/h]** | **Produktanalyse** | | | | | **M/IM^{*}** |
| | | **NM** | **NIM** | **M** | **IM** | **Summe Menthole** | |
| 94 | 173 | 15,352 | 0,020 | 84,261 | 0,204 | 99,837 | 413,0 |

| | | | | | | | |
|---|---|---|---|---|---|---|---|
| NIM = d,1-Neoisomenthol, NM = d,1-Neomenthol, M = d,1-Menthol, IM = d,1-Isomenthol M/IM^{*} = Menthol-Isomenthol-Verhältnis | | | | | | | |

Die Mischung aus Tabelle 3 wird einer fraktionierten Destillation unterworfen (ca. 160 theoretische Böden, Rücklaufverhältnis 40:1. Es wird am Kopf der Kolonne 99,1 % iges (-)-Neomenthol erhalten.

Dieser Herstellweg ist der bislang einzig bekannte technisch praktikable Weg um (-)-Neomenthol herzustellen.

**Tabelle 4**

| Ausgangszusammensetzung: 99,5% DL-Menthol | | | | | | | |
|---|---|---|---|---|---|---|---|
| **T [°C]** | **Eintrag V [g/h]** | **Produktanalyse** | | | | | **M/IM^{*}** |
| | | **NM** | **NIM** | **M** | **IM** | **Summe Menthole** | |
| 90 | 175 | 27,328 | 0,068 | 71,696 | 0,688 | 99,780 | 104,2 |
| 90 | 179 | 26,604 | 0,062 | 72,517 | 0,627 | 99,810 | 115,7 |
| 90 | 179 | 26,443 | 0,050 | 72,798 | 0,536 | 99,827 | 135,8 |
| 90 | 178 | 25,922 | 0,048 | 73,333 | 0,524 | 99,828 | 139,9 |
| 90 | 179 | 26,623 | 0,048 | 72,656 | 0,506 | 99,834 | 143,6 |

**Tabelle 5**

| Ausgangszusammensetzung: 99,5% L-Menthol | | | | | | | |
|---|---|---|---|---|---|---|---|
| **T [°C]** | **Eintrag V [g/h]** | **Produktanalyse** | | | | | **M/IM^{*}** |
| | | **NM** | **NIM** | **M** | **IM** | **Summe Menthole** | |
| 91 | 172 | 15,1 | 0,020 | 84,331 | 0,202 | 99,653 | 417,4 |

Die Mischung aus Tabelle 3 wird einer fraktionierten Destillation unterworfen (ca. 160 theoretische Böden, Rücklaufverhältnis 40:1. Es wird am Kopf der Kolonne 99,3 %iges (+)-Neomenthol erhalten.

Dieser Herstellweg ist der einzig bekannte technisch praktikable Weg um (+)-Neomenthol herzustellen.

## Patentansprüche

**1.** Katalysator enthaltend Ruthenium aufgebracht auf einem Trägermaterial, wobei das Trägermaterial Aluminiumoxid ist, **dadurch gekennzeichnet, dass**
• der Katalysator zumindest ein Erdalkalimetallalkoxylat enthält oder
• durch Umsetzung eines Katalysators, der Ruthenium aufgebracht auf einem Trägermaterial enthält, wobei das Trägermaterial Aluminiumoxid ist, mit zumindest einem Erdalkalimetallalkoxylat erhältlich ist.

**2.** Katalysator nach Anspruch 1, **dadurch gekennzeichnet, dass** das als Trägermaterial eingesetzte Aluminiumoxid eine BET-Oberfläche von mindestens 100 m²/g aufweist.

**3.** Katalysator nach Anspruch 1 oder 2, **dadurch gekennzeichnet, dass** der Gehalt an Ruthenium 0,1 bis 35 Gew. beträgt.

**4.** Katalysator nach einem der Ansprüche 1 bis 3, **dadurch gekennzeichnet, dass** als Erdalkalimetallalkoxylate solche der Formel (I) eingesetzt werden,
(R-O)₂M (I)
in der
R jeweils unabhängig für einen primären, sekundären oder tertiären, cyclischen oder acylischen, verzweigten oder unverzweigten Cl bis C20-Alkylrest steht, der gegebenenfalls durch Aryl, C₁-C₄-Alkoxyl oder C₆ bis C₁₄-Aryloxy weiter substituiert sein kann und
M für Calcium, Strontium oder Barium steht.

**5.** Katalysator nach Anspruch 4, **dadurch gekennzeichnet, dass** als Erdalkalimetallalkoxylate solche der Formel (I) eingesetzt werden in der
M für Barium steht.

**6.** Katalysator nach einem der Ansprüche 1 bis 5, **dadurch gekennzeichnet, dass** die Menge des Erdalkalimetallalkoxylates das zur Herstellung des erfindungsgemäßen Katalysators eingesetzt wird so gewählt wird, dass das molare Verhältnis von Ruthenium zu Erdalkalimetall 30:1 bis 1:30 beträgt.

**7.** Verwendung der Katalysatoren nach einem der ansprüchwe 1 bis 6 zur Isomerisierung von Stereoisomeren des Menthols oder Mischungen solcher Stereoisomere.

**8.** Verfahren zur Isomerisierung von Stereoisomeren des Menthols oder Gemischen solcher Stereoisomeren in Gegenwart eines Katalysators nach einem der Ansprüche 1 bis 6.

**9.** Verfahren nach Anspruch 8, **dadurch gekennzeichnet, dass** d,1-Menthol durch katalytische Isomerisierung von Stereoisomeren des Menthols oder Mischungen dieser Stereoisomere hergestellt wird.

**10.** Verfahren nach Anspruch 8, **dadurch gekennzeichnet, dass**
(+)-Menthol (D-Menthol) zu (-)-Neomenthol oder
(-)-Menthol (L-Menthol) zu (+)-Neomenthol oder
(+)-Isomenthol zu (-)-Neoisomenthol oder
(-)-Isomenthol zu (+)-Neoisomenthol
Isomerisiert wird.

**10.** Verfahren nach einem der Ansprüche 8 bis 10, **dadurch gekennzeichnet, dass** es kontinuierlich durchgeführt wird.

**11.** Verfahren nach Anspruch 10, **dadurch gekennzeichnet, dass** die Katalysatorbelastung bei 0,005 bis 5 kg Ausgangsprodukt pro Liter Katalysator und Stunde [kg/l*h] liegt.

**12.** Verfahren nach einem der Ansprüche 8 bis 10, **dadurch gekennzeichnet, dass** es bei einem Gesamtdruck von 25 hPa bis 30 MPa durchgeführt wird.
